# EUROPEAN PATENT APPLICATION

(11) **EP 0 920 874 A1**
(43) Date of publication of application: **09.06.1999**
(21) Application number: 97203823.6
(22) Date of filing: 05.12.1997
(51) Int. Cl.: A61L 15/28, A61L 15/60, C08B 31/18, C08B 31/00, C08B 37/00

(54) **Superabsorbent material and method for producing said material**

(71) Applicant: SCA Mölnlycke, 3704 HE Zeist (NL)
(72) Inventor: Besemer, Arie Cornelis, 3958 CC Amerongen (NL); Thornton, Jeffrey Wilson, 1272 AL Huizen (NL)
(74) Representative: de Bruijn, Leendert C.

(57) **Abstract**

The invention provides a process of producing a superabsorbent polysaccharide derivative by oxidation of a α-glucan, glucomannan or galactomannan to introduce aldehyde functions, the oxidised polysaccharide subsequently being reacted with sulphur dioxide or an equivalent thereof to produce a hydroxysulphonated polysaccharide. The polysaccharide may be crosslinked before or after the oxidation. The preferred polysaccharide is starch or guar.

## Description

The present invention relates to a superabsorbent material based on poly-saccharides and to a method of producing such material. A superabsorbent material is defined as a material capable of absorbing at least 10 times its own weight of water.

Superabsorbent materials of various types are known in the art. Examples are crosslinked polyacrylates and polysaccharides grafted with polyacrylates. A problem related to the use of such superabsorbent materials is that such materials are normally based on non-renewable and/or non-compostable raw materials. Consequently, there is a need for superabsorbent materials which are wholly based on renewable raw materials, such as polysaccharides, and are degraded by micro-organisms or other natural agents after being disposed of.

EP-A-489424 discloses modified starch compositions useful as absorbable dusting powders, obtained by slight hypochlorite oxidation of starch resulting in the presence of 0.05 - 0.5 wt.% of carboxyl groups (= 0.002 - 0.018 carboxyl groups per monosaccharide unit), followed by partial crosslinking with 0.05 - 1.1 wt.% of phosphorus oxychloride. The powders obtained according to this prior art are not suitable for use in absorbent articles such as diapers, sanitary napkins and the like due to the fact that they do not swell to a significant extent.

Kulicke et al (*Starch*/*Stärke*, **41** (1989) 140-146; *ibid*. **42** (1990) 134-141) have studied the effect of crosslinking with epichlorohydrin and sodium trimetaphosphate, respectively, on the absorption capacity of starch, amylopectin and commercial oxidised starch, which contains carbonyl (aldehyde or ketone) groups and carboxylic acid groups. The degree of oxidation of such oxidised starch is presumably lower than 10%. The purpose of using oxidised starch was to obtain thin boiling starch (lower molecular weight). According to these authors, the absorption capacity decreases with increased crosslinker concentration and the absorption capacity of crosslinked oxidised starch is higher than that of crosslinked native starch. Although a considerable swelling capacity in pure water was reported, the swelling capacity of the crosslinked polymers in water containing sodium chloride or other salts drops to about 15 g per g polymer.

Hydroxysulphonation of starch is known from US patents 2,880,236 and 3,098,869. WO 97/21733 discloses sulphonated cellulose having absorbent properties.

A new type of superabsorbent material based on natural and/or compostable polysaccharides has now been found, which can be obtained by oxidising a polysaccharide to an extent which corresponds to an average presence of at least 0.1 aldehyde group per monosaccharide unit, reacting the aldehyde groups with sulphur dioxide or an equivalent thereof and preferably slightly crosslinking the oxidised polysaccharide prior to and/or after the oxidation.

The polysaccharides to be used according to the present invention are in particular α-glucans like starch, galactomannans like guar gum (guaran) and locust bean gum, and glucomannans including e.g. xanthan gum. Starch and guar are preferred for economic reasons, but other polysaccharides are also suitable, provided their monosaccharide units are sufficiently susceptible to oxidation and crosslinking. If crosslinking is preformed, the polysaccharide can also be of other types, such as β-glucans including cellulose and chitin. The chain length of the polysaccharides is important although there is no critical minimum for the molecular weight. In general, polysaccharides having a molecular weight of more than 1,000 are preferred. A molecular weight above about 25,000 may have a positive effect on the properties of the sulphonated product. The degree of substitution of sulphonation is preferably between 0.1 and 2.0, in particular between 0.2 and 1.0 -CH(OH)SO₃M groups per monosaccharide unit. Herein M represents hydrogen, an ammonium group or a metal, especially an alkali metal such as sodium or potassium.

Oxidation of polysaccharides to aldehyde functions, in particular oxidation of starch, is well-documented. The oxidation can be mainly directed at a vicinal diol function present in the monosaccharide rings, such as the C₂-C₃-site in anhydroglucose units. This results in cleavage of the monosaccharide units with the production of dialdehyde and/or dicarboxyl functions. Periodic acid is the oxidising agent of choice for this type of reaction, although other agents such as manganese oxides and lead tetraacetate can also be used. WO 95/12619 describes an improved oxidation of starch with periodic acid, resulting in dialdehyde starch with extensive regeneration of a periodic acid.

Oxidation of polysaccharides can also be focused at primary hydroxyl groups, like the 6-hydroxyl group in the anhydroglucose units of glucans. An improved oxidation of the 6-hydroxyl groups in starch, using a hypohalite in the presence of a di-tert-alkylnitroxyl catalyst, in particular tetramethylpiperidine-N-oxyl (TEMPO), is disclosed in WO 95/07303. This oxidation is believed to proceed via the aldehyde stage, resulting in a mixture of aldehyde and carboxylic acid functions.

In polysaccharides containing terminal galactose units, such as galactomannans (guar and locust bean gum), the aldehyde functions can also be introduced enzymatically, using galactose oxidase (EC 1.1.3.9).

The aldehyde functions thus obtained are subsequently reacted with sulphur dioxide or its equivalents. Such equivalents comprise sulphite, bisulphite, meta-bisulphite and the like salts. Sulphur dioxide can be used as a gas or as a solution. This reaction results in addition of sulphurous acid, i.e. the function -CHO is converted to the function -CH(OH)SO₃H or its salts.

In the case of dialdehyde starch (and its equivalents of other polysaccharides), all the aldehyde functions can be transformed to sulphonic acid groups, if desired. Alternatively, part of the aldehyde functions can be further oxidised to carboxyl groups, using e.g. chlorite, and the remainder is reacted with sulphur dioxide or an equivalent thereof. These reactions result in products with both carboxyl and sulpho groups, which constitute a preferred embodiment of the invention.

In the case of TEMPO-catalysed oxidation, the aldehyde functions are sulphonated, which again results in a product having both carboxyl and sulpho groups. This sulphonation not only provides a product with effective absorbing properties, but also provides a simpler and useful manner of removing the aldehyde groups, which otherwise would have to be reduced to methylol groups using e.g. borohydrides.

The degree of oxidation is such that more than 1.0 out of each 10 monosaccharide units (whether or not carboxyalkylated) is oxidised to produce an aldehyde/carboxyl or dialdehyde-monosaccharide unit.

Further derivatisation of the oxidised and sulphonated glycan is also possible. Such further derivatisations include e.g. maleic/succinic esterification or equivalent treatment. This also results in a product having similar absorbing properties.

The oxidised polysaccharide is preferably previously or subsequently reacted with a crosslinking agent. Crosslinking agents are reagents containing two or more functions capable of reacting with a hydroxyl group, resulting in intra- and intermolecular bonds between different mono-saccharide units. Suitable crosslinking agents include divinyl sulphone, epichlorohydrin, diepoxybutane and diglycidyl ethers, diisocyanates, trimetaphosphates, phosphoryl chloride, and mixed anhydrides. With dialdehyde polysaccharides, diamines can also be used as cross-linking groups, provided that relatively low levels of crosslinking are performed so as to leave sufficient aldehyde groups for sulphonation. Crosslinking of starch and other polysaccharides is well-known in the art. A description of crosslinking agents and reaction conditions can be found e.g. in "*Starch Derivatives: Production and Uses*" by M.W. Rutenberg and D. Solarek, Acad. Press Inc., 1984, pages 324-332.

The crosslinking before or after oxidation to the desired level of crosslinking can be achieved by reacting the polysaccharide with 0.1-20 mol.% of crosslinking agent, preferably 0.2-10 mol.% and in particular 0.5-5 mol.%. Optimum crosslinking conditions depend on the particular polysaccharide and on the particular crosslinking agent. Reagents such as diglycidyl ethers are usually reacted under acidic conditions, whereas most other reagents are reacted under neutral or alkaline conditions. Reaction conditions and working-up procedures are described e.g. in US 4,582,865. Work-up may be done by drawing off the aqueous solution of cross-linking agent from the gelatinised polysaccharide, drying the latter under air, under reduced pressure and/or in a drying oven, optionally using water-miscible and/or volatile solvents such as methanol, ethanol, isopropanol, acetone, dioxane or tetrahydrofuran, followed e.g. by freeze-drying, spray-drying, vacuum-drying or other appropriate technique. Crosslinking preferably results in a gel, that may precipitate from the reaction medium. Some pre-crosslinked starches are commercially available and can be used in the present invention.

Prior crosslinking is preferred over post-crosslinking. Introduction of sulphonic acid groups in a pre-crosslinked periodate oxidised starch gives a gel within 15 minutes.

The superabsorbent polysaccharide according to the invention has useful absorption characteristics, especially of body fluids which contain various salts and non-ionic substances. The product is particularly suitable for the production of absorbent hygiene articles, such as diapers, sanitary napkins and the like. Such articles can be produced entirely on the basis of the polysaccharides according to the invention, but they can also contain conventional absorbent materials, such as cellulose pulp in addition to the absorbents according to the invention.

### Example 1

To a suspension of 500 mg (90% DO, degree of oxidation) dialdehyde starch in 25 ml water, 5 ml of a solution of 39% (w/v) sodium bisulphite was added. The mixture was stirred at room temperature. During the reaction the solid material dissolved and the viscosity of the solution increased. After 3 days all material was dissolved. The solution was then poured into methanol (1:3) and the sulphonated starch was collected by filtration or centrifugation, washed with methanol and dried at room temperature.

### Example 2

To a suspension of 500 mg (45% DO, degree of oxidation) dialdehyde starch, crosslinked with 0.5 % epichlorohydrin, 25 ml of a solution of 39% (w/v) sodium bisulphite was added. After 15 minutes swelling of the material was observed, and after 30 minutes the water was completely absorbed due to gel formation. The material was isolated as described in example 1.

### Example 3

Starch (10 g, corresponding to 62 mmol anhydroglucose units), suspended in 14% sodium sulphate solution, was crosslinked with 0.10 (10 mg), 0.15(15 mg) or 0.20 wt.% (20 mg) of epichlorohydrin (see US 2,929,811). After 20 hours, the product was isolated by filtration and washed several times with water to remove all salts. The product was treated with 96% ethanol and acetone and dried in a vacuum oven. The dried material was suspended in water (250 ml), 6.6 g or 10.6 of sodium periodate was added and the mixture was stirred for 20 h at 5°C in the dark to obtain an oxidised product with a DO of 50% or 80%, respectively. The product was collected by filtration and washed with water until the filtrate was free of periodate and iodate. A dry product was obtained by freeze-drying. In a suspension of 1 g of the dry material in 20 ml of water, 1.6 ml (in the case of DO of 50%) or 2.3 ml (DO of 80%) of 39% sodium bisulphite solution was added. After standing, a gel was formed gradually (visible within 15 minutes). The product was precipitated in 96% ethanol and then collected and dried. The yield was 1.7 g (DO of 50%) and 2.2 g (DO of 80%), respectively. After isolation of the product, its free swelling capacity (FSC) in synthetic urine (2 hours of swelling) was measured (w/w). The composition of the synthetic urine is as follows:
300 mM of urea
60 mM of KCl
130 mM of NaCl
3.5 mM of MgSO₄
2.0 mM of CaSO₄.2H₂O
1 g/l of Triton X-100 solution of 0.1% (Riedel de Haen); in deionised water.

The results are summarised in table 1.

**Table 1**

| **FSC results of pre-crosslinked dialdehyde starch treated with bisulphite.** | | | |
|---|---|---|---|
| sample | % crosslinking | DO | FSC after 2 h. |
| 1 | 0.10 | 50 | 19.0 |
| 2 | 0.10 | 80 | 22.6 |
| 3 | 0.15 | 50 | 16.1 |
| 4 | 0.15 | 80 | 18.2 |
| 5 | 0.20 | 50 | 12.6 |
| 6 | 0.20 | 80 | 15.9 |

## Claims

1. Use of a hydroxysulphonated α-glucan, glucomannan or galactomannan as a water-absorbing material.

2. Use of a crosslinked, hydroxysulphonated polysaccharide as a water-absorbing material.

3. Process of producing a superabsorbent polysaccharide derivative by oxidation of a polysaccharide, characterised in that a polysaccharide is crosslinked with 0.001-0.2 equivalents of crosslinking agent per monosaccharide unit and, before or after said crosslinking, the polysaccharide is oxidised to introduce at least 0.1 aldehyde functions per monosaccharide unit and the crosslinked, oxidised polysaccharide is subsequently reacted with sulphur dioxide or an equivalent thereof to produce a hydroxysulphonated polysaccharide.

4. Process according to claim 3, wherein the polysaccharide is selected from α-glucans, glucomannans and galactomannans.

5. Process according to claim 3 or 4, wherein the polysaccharide is crosslinked with epichlorohydrin, a trimetaphosphate, phosphoryl chloride, divinyl sulphone, diglycidyl ether, a diisocyanate, a mixed anhydride or a diamine.

6. Process according to any one of claims 3-5, wherein said hydroxy-sulphonated polysaccharide contains 0.1-2 sulpho groups per monosaccharide unit.

7. Process according to any one of claims 3-6, wherein the polysaccharide is oxidised using periodic acid.

8. Process according to any one of claims 3-6, wherein the polysaccharide is oxidised using hypochlorite in the presence of a di-tert-alkylnitroxide.

9. Sulphonated crosslinked polysaccharide, containing between 0.1 and 2 -CH(OH)(SO₃M) groups, wherein M is H or a metal, per monosaccharide unit.

10. Sulphonated polysaccharide according to claim 9, which is a α-glucan, glucomannan or galactomannan, especially starch or a starch derivative.

11. Sulphonated polysaccharide according to claim 9 or 10, further containing between 0.1 and 1.0 carboxyl groups per monosaccharide unit.
